# EUROPEAN PATENT SPECIFICATION

(11) **EP 2 076 412 B1**
(45) Date of publication and mention of the grant of the patent: **14.11.2012**
(21) Application number: 07754121.7
(22) Date of filing: 29.03.2007
(51) Int. Cl.: B60S 1/02, A61B 1/00, A61B 1/12

(54) **METHOD AND MEANS TO BLOW FLUID OVER-AND KEEP CLEAN - THE TRANSPARENT SURFACE OF AN OPTICAL ELEMENT**
VERFAHREN UND MITTEL ZUM BLASEN VON FLÜSSIGKEITEN ÜBER UND SAUBER HALTEN DER TRANSPARENTEN OBERFLÄCHE EINES OPTISCHEN ELEMENTS
PROCÉDÉ ET MOYEN POUR SOUFFLER SUR ET MAINTENIR LA SURFACE TRANSPARENTE D'UN ÉLÉMENT OPTIQUE

(30) Priority: 03.04.2006 US 396779
(43) Date of publication of application: 08.07.2009
(73) Proprietor: Bral, Pourang, Passaic NJ 07055 (US)
(72) Inventor: Bral, Pourang, Passaic NJ 07055 (US)
(74) Representative: WP Thompson
(86) International application number: PCT/US2007/007551
(87) International publication number: WO 2008/018924

(56) References cited:
- JP-A- 8 126 605
- US-A- 759 622
- US-A- 897 290
- US-A- 1 357 887
- US-A- 5 386 817
- US-A- 5 575 756
- US-B1- 6 248 060

## Description

US 5,386,817 discloses a protective covering for a medical instrument, such as an endoscope. The covering includes an elongated hollow sheath having a wall of flexible material. The sheath is substantially gas and water impervious. The sheath further includes auxiliary access tubes associated with the sheath for providing a variety of functions, such as instrument manipulation, and fluid removal. The distal end of the sheath is provided with a cap having an optically clear window to allow the lens portion of the medical instrument to operate.

According to an aspect of the present invention, there is provided an optical element including a plurality of transparent surfaces which are used to capture a plurality of images, said optical element being covered in a cover including a plurality of see through surfaces at least part of which or the area adjacent to them is flexible and is positioned opposite a plurality of said transparent surfaces, said optical element or said cover further including a plurality of channels for directing compressed gas from an outside source to said plurality of see-through surfaces so that when compressed gas blows over at least one said see through surface, said see through surface is pressed against said transparent surface, enhancing adherence and avoiding bubbles between said see through surface and said transparent surface for better imaging quality by said optical element.

According to another aspect of the present invention, there is provided a method of removing debris or liquids that would otherwise collect around objects that are to be viewed and/or imaged by an optical element for enhanced viewing and imaging and at the same time keeping transparent surfaces of said optical element free from flying debris or liquids that would otherwise stick to a plurality of said transparent surfaces and block the view of the objects to be viewed and/or imaged, said method comprising: using an optical element including a plurality of transparent surfaces which are used to capture a plurality of images, said optical element being covered in a cover including a plurality of see through surfaces at least part of which or the area adjacent to them is flexible and is positioned opposite a plurality of said transparent surfaces, said optical element or said cover further comprising a plurality of channels for implementing the step of directing compressed gas from an outside source to a plurality of said see through surfaces so that when compressed gas blows over at least part of at least one said see through surface, said see through surface is pressed against said transparent surface, thereby enhancing adherence and avoiding bubbles between said see through surface and said transparent surface for better imaging quality by said optical element.

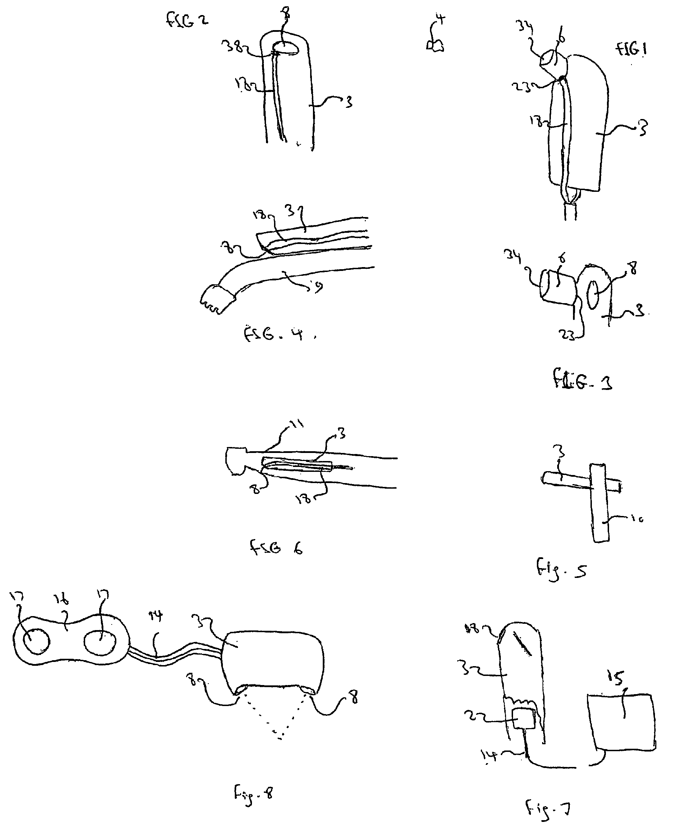

The invention comprises of a means and a method to keep clean a plurality of transparent surfaces 8, through which a plurality of images are captured by an optical element 3 which may be an optical sensor or a camera, where there is flying debris or liquids that can potentially stick to a plurality of the transparent surfaces 8 and clog the view of said optical element 3, Fig. 2. A plurality of channels 18 transfer and direct fluid such as compressed air from an outside source to blow over and keep a plurality of the transparent surfaces 8 free of debris or liquids. In a specific example of the invention, a plurality of said channels 18 direct fluid such as compressed air through a plurality of foramens 38 located at a distance close enough to-- and blowing fluid such as compressed air in the direction of--a plurality of transparent surfaces 8 to effectively blow away the flying debris or liquids from the plurality of transparent surfaces 8. In another example of the invention, a plurality of channels 18 direct fluid such as compressed air through a plurality of pores 23 to a plurality of tubes 6, each tube 6 including an inner opening 33 facing a plurality of transparent surfaces 8 and an outer opening 34 through which fluid such as compressed air exits the tube 6, Fig. 1 and Fig. 3. Furthermore, a plurality of tubes 6 allow a plurality of images to pass through and reach the transparent surface 8 while the exiting fluid such as compressed air blows away any flying debris or liquids and prevents it from reaching and sticking the transparent surface 8.

Said optical element 3 can, for example, be used to monitor the hard and soft tissues, along with a variety of activities performed by an operator such as a healthcare provider, in or near the mouth.

In a specific example of the invention, said optical element 3 can be mounted separably or permanently on an object used in or near his/her mouth such as a saliva ejector 9, Fig 4, or a bite block 10 used to keep his/her mouth open, Fig.5. Said optical element 3, by way of example, can also be attached to the hand piece 11 or drill the operator uses, Fig. 6. In another example of the invention, said optical element 3 can be separably mounted on the body of the operator, for example on his head, to allow the operator to adjust his/her viewing angle at will by positioning his head in a desired manner.

In a specific example of the invention, said optical element 3 includes a plurality of imagers 2, such as digital imaging arrays or modules, that are connected through a plurality of electrical wires 14 or wirelessly and transmit their data to a monitor 15 where the operator can view the field of operation, Fig. 7. In a specific example of the invention, said optical element 3 includes two said transparent surfaces 8 focusing on the same field of operation each slightly from a different angle, Fig. 8, each directing its image of the field of operation to an imager 2. In a specific example of the invention, the data from said imagers 2 are transmitted directly or indirectly to a viewing box 16 comprising two visors 17 corresponding to the two eyes of the operator. This way the image captured by each said imager 2 is eventually viewed by one of the operator's eyes. Said two transparent surfaces 8 can be positioned such that the fields of their vision converge so as to afford the operator to have depth perception of the operation field when viewing through said visors 17. In a specific example of the invention, said viewing box 16 is wide enough to shield the operator's face from debris or liquids flying from the mouth of the person being operated on.

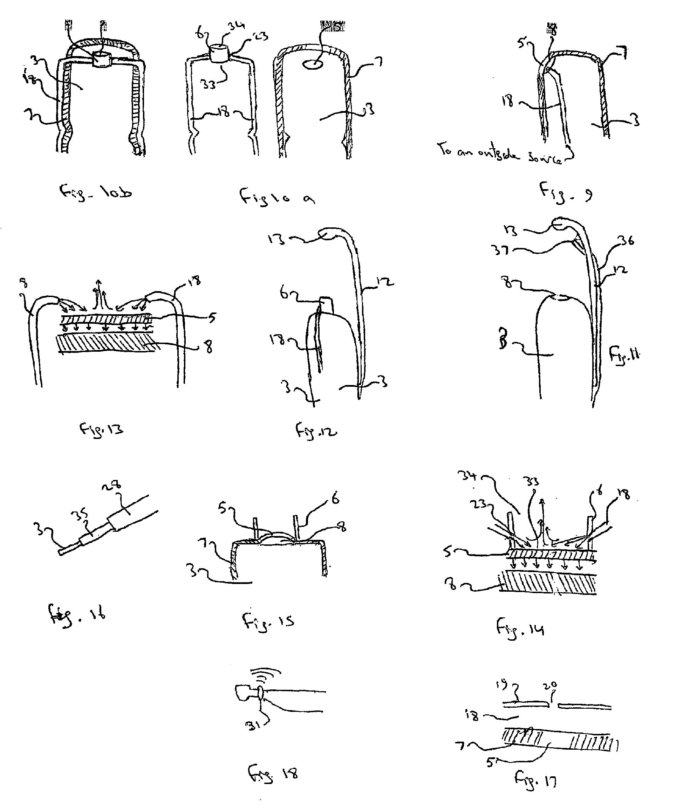

In a specific example of the invention, said optical element 3 can be permanently or removably contained in a cover 7 which may be autoclavable and/or disposable. Said cover 7 will have adequate see-through surface 5 opposite said transparent surface 8 to allow image capture by said optical element 3, Fig.9. A plurality of channels 18 transfer and direct fluid such as compressed air to blow over said see-through surface 5 to keep said see-through surface 5 free of debris or liquids. Said plurality of channels 18 and/or a plurality of said tubes 6 are, in one example of the invention, included as part of said cover 7 to obviate the need for handling several loose components. The operator merely places the cover 7 onto the optical element 3; the plurality of channels 8 and/or a plurality of tubes 6 are then perforce positioned accurately on the optical element 3. In a specific example of the invention, a plurality of said channels 18, running across said cover 7, are not easily bendable so as to give stiffness to and stabilize said cover 7 when placed over said optical element 3. The advantage of such a design is 1) to avoid bubbles between said transparent surface 8 and said see-through surface 5 because the cover 7 in held in position unable to move, and 2) to hold a plurality of said tubes 6 and/or said foramens 38, which are secured on said cover 7, in position with respect to the optical element 3. In a specific example of the invention, said cover 7 includes an outer wall 19 with a plurality of orifices 20, Fig. 17. Said outer wall 19 is positioned between the flying debris and said see-through surface 5, generally parallel to said see-through surface 5. Said plurality of orifices 20 are positioned opposite said imagers 2 so as to allow said imagers 2 to view the field through a plurality of said orifices 20. Said channels 18 direct fluid such as compressed gas out of said orifices 20 to prevent debris or liquids from entering said orifices 20 and to wash or blow away any debris or liquids that do enter said orifices 20; whereas light from the field of operation enters through said orifices 20, passes through a plurality of said see-through surfaces 5, a plurality of transparent surfaces 8, and reaches and is captured by said imagers 2.

In a specific example of the invention, a plurality of tubes 6, with an inner opening 33 and an outer opening 34 are positioned on said see-through surface 5 of said cover 7 with said inner opening 33 facing said see-through surface 5 which is facing said transparent surface 8, in a manner that an image can travel through said plurality of tubes 6, said see-through surface 5, said transparent surface 8, and to a plurality of said imagers 2, Fig. 10a and Fig. 10b. A plurality of said channels 18, together with a plurality of said tubes 6, form an integral part which is then removably fastened over said cover 7 covering said optical element 3. This design helps stabilize said cover 7 over said optical element 3 and avoid bubbles between a plurality of transparent surfaces 8 and said see-through surface5. This design is especially helpful to avoid bubbles between a plurality of transparent surfaces 8 and a plurality of see-through surfaces 5 if at least part of the cover 7 is flexible. A plurality of said channels 18 direct fluid such as compressed air into a plurality of said tubes 6 through a plurality of pores 23. Fluid such as compressed air which is introduced into a plurality of said tubes 6 by means of a plurality of said channels 18 exits a plurality of said tubes 6 through said outer opening 34 and prevents flying debris or liquids from entering said plurality of tubes 6, sticking to said see-through surface 5, and blocking the image from reaching said imagers 2.

In a specific example of the invention, a plurality of attachments 12 install a plurality of mirrors 13 to the optical element 3; said mirrors 13 reflecting the image of the field of operation from a plurality of different angles onto the transparent surface 8 of said optical elements 3 to be captured by a plurality of said imagers 2, Fig. 11. In a specific example of the invention, fluid such as compressed air flows through a plurality of ducts 36 that run near a plurality of said mirrors 13 and is blown onto a plurality of said mirrors 13, ther :by cleaning them from debris or liquids. A plurality of said ducts 36, for example, are supported by and run along a plurality of said attachments 12 to a location near a plurality of said mirrors 13 before blowing compressed air onto a plurality of said mirrors 13. In another example of the invention, a plurality of said attachments 12 are hallow. Compressed air flows through a plurality of said attachments 12 exiting through a plurality of nozzles 37 located at a distance short enough from-and blowing onto--a plurality of said mirrors 13 to effectively blow the debris or liquids away from a plurality of said mirrors 13. In a specific example of the invention, fluid such as compressed air, originating from a plurality of said tubes 6 and/or a plurality of said orifices 20, is blown onto a plurality of said mirrors 13, thereby cleaning them from debris or liquids. In a specific example of the invention, at least part of the surface of at least one said mirror 13 is curved, for example concavely shaped, so that the view of more of the field of operation is reflected through said plurality of mirrors 13.

The available intra-oral optical elements today are generally covered in a disposable clear plastic sheath for sanitation purposes. A problem associated with these sheaths is they loose adherence to a transparent surface 8 sometimes during the operation, forming a bubble between these sheaths and a transparent surface 8; and the image shown on the monitor 15 or said viewing box 16 will become blurry. This problem is tackled with in a specific example of the invention as follows. Fluid such as compressed air is blown over said see-through surface 5 of said cover 7 by means of a plurality of said channels 18, pressing and helping maintain the adherence of said see-through surface 5 to said transparent surface 8, avoiding bubbles between said see-through surface 5 and said transparent surface 8, and allowing a more clear image to be viewed in the monitor 15 or said viewing box 16, Fig. 13. This is of special importance when the cover 7 is generally elastic and flexible at least in the area of a see-through surface 5 facing a transparent surface 8. However, even if the cover 7 is rigid, this design enhances the adherence of the transparent surface 8 and the see-through surface 5, and therefore improves the clarity of the image.

In a specific example of the invention, a plurality of said tubes 6 containing at least one said pore 23 are positioned on said see-through surface 5. Fluid such as compressed air is allowed to flow into a plurality of said tubes 6 through at least one said pore 23, Fig. 14. Through said inner opening 33 of at least one said tube 6, the fluid such as compressed air will press said see-through surface 5 to said transparent surface 8 maintaining adherence between same before exiting from said outer opening 34 of said tube 6 and preventing debris or liquids to enter said tube 6 and adhere to said see-through surface 5.

In a specific example of the invention, at least one said tube 6 physically contacts and presses said see-through surface 5 against said transparent surface 8, supporting the adherence between a said see-through surface 5 and a said transparent surface 8, Fig. 15.

In a specific example of the invention, said orifice 20 or said outer opening 34 of said tube 6 is small enough to prevent unwanted light rays-that would distort the image-from reaching said imagers 2. The small said orifice 20 or said opening would effectively act as a lens.

In a specific example of the invention, a plurality of said imagers 2 are surrounded at least partially by a fluid such as compressed air before it exits at least one said orifice 20 and/or at least one said tube 6. Said surrounding fluid, e.g.: compressed air, acts as a cooling medium to ventilate said imager 2 and prevent it from heating up above its operating temperature. For example, the image sensor module, VS6502V015, manufactured by ST Microelectronics, has a max. operating temperature of 40 degrees C. If the module is used for example in the oral cavity with a temperature of about 37 degrees C for an extended period of time, it is conceivable to see how the temperature in the vicinity of the module may increase above 40 degrees C due to the heat generated by the body of the person operated on and the lighting source. In this and similar scenarios, the ventilation offered by compressed air that surrounds said imager 2 can be important.

In a specific example of the invention, a clear moving surface 26 is provided over said transparent surface 8 to shield said transparent surface 8 from flying debris or liquids. Said moving surface 26 can be rotating for example. A squeegee 27, which is stationary for example and is positioned on said moving surface 26, wipes said moving surface 26 clean of debris or liquids as it rotates so the debris or liquids does not block the view of said imagers 2. In a specific example of the invention, said moving surface 26 rotates very fast to effect removal of debris or liquids through the process of centrifuging.

In a specific example of the invention, said viewing box 16 is movably fastened to the operator's chair or to a wall mount so as to allow the operator to adjust the position of said viewing box 16 and thereby maintain a comfortable position on the chair while operating. This feature minimizes the back pain and discomfort experienced by many dentists that would otherwise have to bend excessively in sometimes uncomfortable-and unhealthy-positions to focus on the field of operation.

In another example of the invention, said optical element 3 is supported and held in position on a mount 28. Said optical element 3 being supported and held in position on a mount 28 will allow the operator to conveniently position said optical element 3 in a desired angle for enhanced viewing, Fig. 16. In a specific example of the invention, said optical element 3 is separable from said mount 28 and can be manually held by the operator in a desired position. In another example of the invention, said optical element 3 is mounted separably or otherwise on an extendable, telescopic arm 35 which is supported by a said mount 28. This will allow the operator to bring a said optical element 3 near the operating field such as in or near the patient's mouth and maintain and stabilize said optical element 3 in a desired position hands free.

In a specific example of the invention, a said cover 7 which is generally see-through and is fabricated as a fitting fits over and covers said optical element 3. A plurality of said channels 18 run along said cover 7, transferring fluid such as compressed air from an outside source to a plurality of foramens 38 that allow compressed air to exit to the outside of said channels 18. Said foramens 38 are located at a distance short enough from said see-through surface 5 facing said transparent surface 8 so that compressed air blowing from a plurality of said foramens 38 can blow over and effectively clean and blow away any debris or liquids that may otherwise stick to said see-through surface 5. In order for a plurality of said foramens 38 and /or tubes 6 to accurately blow air in a desired direction, e.g. onto said see-through surface 5 or away from the see-through surface 5, they must be firmly held in position. One way to hold said foramen 38 and/or a tube 6 in position is provided by a said cover 7 that is rigid at least partially around said foramen 38 and stabilizes its position. Another example is provided by a plurality of said channels 18 that are rigid and stabilize a plurality of said foramens 38 that are located at some point on said channels 18. A third example is to fabricate at least one said channel 18 from generally flexible material. When compressed air is introduced into a said channel 18, said channel 18 expands and becomes stiff and stabilizes the position of a plurality of said foramens 38.

Also disclosed, is a method of blowing away any debris or liquids that collect around the field of vision. In a preferred example of the invention, fluid such as compressed air exiting a plurality of tubes 6 blows on the field being imaged and removes any liquids and/or debris or liquids that may have collected there. In a specific example of the invention, fluid such as compressed air also directs any such liquids and/or debris to a location where such liquids and/or debris is disposed of, for example by a saliva ejector, while an optical element 3 is imaging the field of operation. In certain surgical and/or dental procedures, blood, saliva, debris and/or liquids introduced by the operator-for example, water used in dental hand pieces and cavitron tips-collect around and obstruct the view of the field of operation. In the method disclosed in the invention, a said optical element 3 along with a plurality of said tubes 6 that blow compressed air are used to 1) prevent the view of said optical element 3 from being clogged by keeping clean the see-through surface 5 and/or the transparent surface 8, 2) blow compressed air onto the field of operation, blowing away and, for example, guiding such liquids to a location where they are disposed, for example by a suction tip positioned where said liquids are guided to; and 3) image the field of operation as disclosed above.

Even though in the examples of the invention disclosed above said imager 2 captures the image of the objects to be viewed directly through a said transparent surface 8, it is clear to one skilled in the art that the image of the objects to be viewed-- after passing through said transparent surface 8-- can be reflected through a plurality of mirrors and/or a plurality of optic fibers (not shown), and directed to a plurality of said imagers 2 which would then be located at a distance to said transparent surface 8.

In a related invention, a homing device 31 is separably or permanently fixed on the instruments used by an operator such as a dentist, for example on a hand piece 11, explorer, perio probe, cavitron tips, etc... Said homing device 31 emits a signal, for example of a radio-magnetic frequency, Fig. 18, which can be detected by a processor. Once the image is captured by an optical element 3 and processed by a processor, the processor recognizes said homing device 31 and may be instructed to focus on the area in the immediate vicinity of said homing device 31. This invention allows the operator to automatically focus on and magnify the view of the area in the immediate vicinity of the field of operation where he is using the instrument that carries a said homing device 31. This invention is especially useful when the operator is working on different areas in one session, or when the person operated on is not still. Said homing device 31 communicates to the processor what area the operator is working on. When the operator moves on to the next area, or the person operated on moves and brings the field of vision out of view, the processor automatically readjusts, focuses on, and magnifies the new area for better clarity.

Even though the examples of this invention set forth above relate mostly to the dental field, it is clear to the one skilled in the art that the invented design is also useful in many branches of medicine such as laparoscopy, hysteroscopy, and orthopedic surgery, as well as many industrial applications where the need to stop and clean said transparent surface 8 from debris or liquids is obviated. One skilled in the art will appreciate that the present invention can be practiced by other than the described embodiments, which are presented here for purposes of illustration and not of limitation, and the present invention is limited only by the claims that follow.

## Claims

1. Optical element (3) including a plurality of transparent surfaces (8) which are used to capture a plurality of images, said optical element being covered in a cover (7) including a plurality of see-through surfaces (5) at least part of which or the area adjacent to them is flexible and is positioned opposite a plurality of said transparent surfaces, said optical element or said cover further including a plurality of channels (18) for directing compressed gas from an outside source to said plurality of see-through surfaces so that when compressed gas blows over at least one said see-through surface, said see-through surface is pressed against said transparent surface, enhancing adherence and avoiding bubbles between said see-through surface and said transparent surface for better imaging quality by said optical element.

2. Optical element (3) according to claim 1 in which a plurality of said channels (18) are arranged to lead compressed gas to a plurality of tubes (6) with an inner opening (33) and an outer opening (34), said tubes being positioned on said cover (7) so that said inner opening of said plurality of tubes is located on and faces a said plurality of see-through surfaces (5).

3. Optical element (3) according to claim 2 in which said tubes (6) are so positioned as to direct compressed gas exiting said outer opening (34) onto the field of vision, blowing away the debris or liquids and liquids that would otherwise collect in the field of vision for enhanced imaging of the objects in the field of vision.

4. Optical element (3) as in claim 1 in which said plurality of channels (18) are arranged to direct compressed gas through a plurality of foramens (38) onto a plurality of said see-through surfaces (5).

5. Optical element (3) according to claim 2 in which said plurality of tubes (6) are secured onto and/or are part of said cover (7).

6. A method of removing debris or liquids that would otherwise collect around objects that are to be viewed and/or imaged by an optical element (3) for enhanced viewing and imaging and at the same time keeping transparent surfaces (8) of said optical element free from flying debris or liquids that would otherwise stick to a plurality of said transparent surfaces and block the view of the objects to be viewed and/or imaged, said method comprising: using an optical element including a plurality of transparent surfaces which are used to capture a plurality of images, said optical element being covered in a cover (7) including a plurality of see-through surfaces (5) at least part of which or the area adjacent to them is flexible and is positioned opposite a plurality of said transparent surfaces, said optical element or said cover further comprising a plurality of channels (18) for implementing the step of directing compressed gas from an outside source to a plurality of said see-through surfaces so that when compressed gas blows over at least part of at least one said see-through surface, said see-through surface is pressed against said transparent surface, thereby enhancing adherence and avoiding bubbles between said see-through surface and said transparent surface for better imaging quality by said optical element.

7. A method according to claim 6, comprising directing compressed gas from a plurality of said channels (18) to a plurality of pores (23) and into a plurality of tubes (6) including an inner opening (33) and an outer opening (34), said inner opening facing a plurality of said see-through surfaces (5) and allowing a plurality of images to pass through said tube to be captured by a plurality of said transparent surfaces (8), from which plurality of tubes compressed gas exits through said outer opening and blows onto the objects to be viewed in the field of vision, thereby removing any debris or liquids and liquids that would otherwise collect around those objects and block the view of same.

## Patentansprüche

1. Optisches Element (3), umfassend mehrere transparente Flächen (8), die verwendet werden, um mehrere Bilder zu erfassen, wobei das genannte optische Element in einer Abdeckung (7) abgedeckt ist, die mehrere durchsichtige Flächen (5) umfasst, von denen mindestens ein Teil oder der daran angrenzende Bereich biegsam ist und gegenüber von mehreren der genannten transparenten Flächen positioniert ist, wobei das genannte optische Element oder die genannte Abdeckung weiter mehrere Kanäle (18) umfasst, um verdichtetes Gas von einer äußeren Quelle zu den genannten mehreren durchsichtigen Flächen zu leiten, so dass wenn verdichtetes Gas über mindestens eine genannte durchsichtige Fläche bläst, die genannte durchsichtige Fläche an die genannte transparente Fläche gedrückt wird, wodurch für bessere Bilderfassungsqualität von dem optischen Element zwischen der genannten durchsichtigen Fläche und der genannten transparenten Fläche die Haftung verbessert wird und Blasen vermieden werden.

2. Optisches Element (3) nach Anspruch 1, wobei mehrere der genannten Kanäle (18) dazu angeordnet sind, verdichtetes Gas zu mehreren Röhren (6) mit einer inneren Öffnung (33) und einer äußeren Öffnung (34) zu führen, wobei die genannten Röhren auf der genannten Abdeckung (7) positioniert sind, so dass sich die genannte innere Öffnung der genannten mehreren Röhren auf genannten mehreren durchsichtigen Oberflächen (5) befindet und diesen zugewandt ist.

3. Optisches Element (3) nach Anspruch 2, wobei die genannten Röhren (6) derart positioniert sind, dass sie aus der genannten äußeren Öffnung (34) austretendes verdichtetes Gas auf das Blickfeld leiten und dadurch für die verbesserte Erfassung der Gegenstände im Blickfeld den Schmutz oder die Flüssigkeiten und Flüssigkeiten wegblasen, die sich sonst im Blickfeld ansammeln würden.

4. Optisches Element (3) nach Anspruch 1, wobei die genannten mehreren Kanäle (18) dazu angeordnet sind, verdichtetes Gas durch mehrere Foramen (38) auf mehrere der genannten durchsichtigen Flächen (5) zu leiten.

5. Optisches Element (3) nach Anspruch 2, wobei die genannten mehreren Röhren (6) an der genannten Abdeckung (7) befestigt sind und/oder Teil davon sind.

6. Verfahren zum Entfernen von Schmutz oder Flüssigkeiten, die sich sonst um zu betrachtende und/oder von einem optischen Element (3) zu erfassende Gegenstände ansammeln würden, um die Betrachtung und Erfassung zu verbessern und gleichzeitig transparente Flächen (8) des genannten optischen Elements frei von fliegendem Schmutz oder Flüssigkeiten zu halten, die sonst an mehreren der genannten transparenten Flächen kleben würden und die Sicht auf die zu betrachtenden und/oder erfassenden Gegenstände blockieren würden, wobei das genannte Verfahren Folgendes umfasst: Verwenden eines optischen Elements, umfassend mehrere transparente Flächen, die verwendet werden, um mehrere Bilder zu erfassen, wobei das genannte optische Element in einer Abdeckung (7) abgedeckt ist, die mehrere durchsichtige Flächen (5) umfasst, von denen mindestens ein Teil oder der daran angrenzende Bereich biegsam ist und gegenüber von mehreren der genannten transparenten Flächen positioniert ist, wobei das genannte optische Element oder die genannte Abdeckung weiter mehrere Kanäle (18) umfasst, um den Schritt des Leitens von verdichtetem Gas von einer äußeren Quelle zu mehreren der genannten durchsichtigen Flächen umzusetzen, so dass wenn verdichtetes Gas über mindestens einen Teil von mindestens einer genannten durchsichtigen Fläche bläst, die genannte durchsichtige Fläche an die genannte transparente Fläche gedrückt wird, wodurch für bessere Bilderfassungsqualität von dem optischen Element zwischen der genannten durchsichtigen Fläche und der genannten transparenten Fläche die Haftung verbessert wird und Blasen vermieden werden.

7. Verfahren nach Anspruch 6, umfassend das Leiten von verdichtetem Gas von mehreren der genannten Kanäle (18) zu mehreren Poren (23) und in mehrere Röhren (6), umfassend eine innere Öffnung (33) und eine äußere Öffnung (34), wobei die genannte innere Öffnung mehreren der genannten durchsichtigen Flächen (5) zugewandt ist und zulässt, dass mehrere Bilder durch die genannte Röhre gelangen, um von mehreren der genannten transparenten Flächen (8) erfasst zu werden, wobei aus den mehreren Röhren verdichtetes Gas durch die genannten äußere Öffnung austritt und auf die zu betrachtenden Gegenstände im Blickfeld bläst, wodurch etwaiger Schmutz oder Flüssigkeiten und Flüssigkeiten entfernt werden, die sich sonst um diese Gegenstände ansammeln und den Blick auf dieselben blockieren würden.

## Revendications

1. Elément optique (3) comportant une pluralité de surfaces transparentes (8) qui servent à capturer une pluralité d'images, ledit élément optique étant couvert dans un couvercle (7) comportant une pluralité de surfaces translucides (5) dont au moins une partie ou dont la zone qui leur est adjacente est souple et positionnée en face d'une pluralité desdites surfaces transparentes, ledit élément optique ou ledit couvercle comportant en outre une pluralité de canaux (18) pour diriger un gaz comprimé provenant d'une source extérieure vers ladite pluralité de surfaces translucides de telle sorte que lorsque le gaz comprimé souffle par-dessus au moins une dite surface translucide, ladite surface translucide soit pressée contre ladite surface transparente, rehaussant l'adhérence et évitant les bulles d'air entre ladite surface translucide et ladite surface transparente pour une meilleure qualité d'imagerie par ledit élément optique.

2. Elément optique (3) selon la revendication 1, dans lequel une pluralité desdits canaux (18) est agencée pour conduire un gaz comprimé vers une pluralité de tubes (6) ayant une ouverture interne (33) et une ouverture externe (34), lesdits tubes étant positionnés sur ledit couvercle (7) de telle sorte que ladite ouverture interne de ladite pluralité de tubes soit située sur ladite pluralité de surfaces translucides (5) et lui fasse face.

3. Elément optique (3) selon la revendication 2, dans lequel lesdits tubes (6) sont ainsi positionnés de manière à diriger le gaz comprimé sortant de ladite ouverture externe (34) sur le champ de vision, balayant ainsi les débris ou liquides et les liquides qui autrement s'accumuleraient dans le champ de vision pour une imagerie rehaussée des objets dans le champ de vision.

4. Elément optique (3) selon la revendication 1, dans lequel ladite pluralité de canaux (18) est agencée pour diriger le gaz comprimé à travers une pluralité d'orifices (38) sur une pluralité desdites surfaces translucides (5).

5. Elément optique (3) selon la revendication 2, dans lequel ladite pluralité de tubes (6) est fixée sur ledit couvercle (7) et/ou en fait partie.

6. Procédé d'élimination de débris ou liquides qui autrement s'accumuleraient autour d'objet à visualiser et/ou imager par un élément optique (3) pour une visualisation et une imagerie rehaussées tout en maintenant les surfaces transparentes (8) dudit élément optique dépourvues de débris volants ou liquides qui autrement adhéreraient à une pluralité desdites surfaces transparentes et bloqueraient la vue des objets à visualiser et/ou imager, ledit procédé comprenant : l'utilisation d'un élément optique comportant une pluralité de surfaces transparentes qui servent à capturer une pluralité d'images, ledit élément optique étant couvert dans un couvercle (7) comportant une pluralité de surfaces translucides (5) dont au moins une partie ou dont la zone qui leur est adjacente est souple et positionnée en face d'une pluralité desdites surfaces transparentes, ledit élément optique ou ledit couvercle comportant en outre une pluralité de canaux (18) pour mettre en oeuvre l'étape consistant à diriger un gaz comprimé provenant d'une source extérieure vers une pluralité desdites surfaces translucides de telle sorte que lorsque le gaz comprimé souffle par-dessus au moins une partie d'au moins une dite surface translucide, ladite surface translucide soit pressée contre ladite surface transparente, rehaussant l'adhérence et évitant les bulles d'air entre ladite surface translucide et ladite surface transparente pour une meilleure qualité d'imagerie par ledit élément optique.

7. Procédé selon la revendication 6, comprenant l'orientation d'un gaz comprimé provenant d'une pluralité desdits canaux (18) vers une pluralité de pores (23) et dans une pluralité de tubes (6) comportant une ouverture interne (33) et une ouverture externe (34), ladite ouverture interne faisant face à une pluralité desdits surfaces translucides (5) et permettant à une pluralité d'images de passer à travers ledit tube pour être capturées par une pluralité desdites surfaces transparentes (8), un gaz comprimé sortant de ladite pluralité de tubes à travers ladite ouverture externe et soufflant sur les objets à visualiser dans le champ de vision, éliminant ainsi tout débris ou liquides et liquides qui autrement s'accumuleraient autour de ces objets et bloqueraient leur visualisation.
